# EUROPÄISCHE PATENTSCHRIFT

(11) **EP 1 180 966 B1**
(45) Veröffentlichungstag und Bekanntmachung des Hinweises auf die Patenterteilung: **19.05.2004**
(21) Anmeldenummer: 99950672.8
(22) Anmeldetag: 08.10.1999
(51) Int. Cl.: A61B 5/0416, H01R 4/24

(54) **ELEKTRISCHE SCHNEIDKONTAKTIERUNG, INSBESONDERE FÜR MEDIZINISCHE EINMALARTIKEL WIE FETALE SKALP-ELEKTRODEN**
INSULATION PIERCING ELECTRIC CONTACT, ESPECIALLY FOR MEDICAL DISPOSABLE ARTICLES SUCH AS FETAL SCALP ELECTRODES
DISPOSITIF ELECTRIQUE AUTODENUDANT DE MISE EN CONTACT, NOTAMMENT POUR ARTICLES JETABLES A USAGE MEDICAL, TELS QUE DES ELECTRODES POUR CUIR CHEVELU DE FOETUS

(30) Priorität: 22.04.1999 EP 99107961
(43) Veröffentlichungstag der Anmeldung: 27.02.2002
(73) Patentinhaber: Koninklijke Philips Electronics N.V., 5621 BA Eindhoven (NL)
(72) Erfinder: SCHMID, Alfons, D-71034 Böblingen (DE)
(74) Vertreter: Schouten, Marcus Maria
(86) Internationale Anmeldenummer: PCT/EP1999/007546
(87) Internationale Veröffentlichungsnummer: WO 2000/064340

(56) Entgegenhaltungen:
- EP-A- 0 131 705
- EP-A- 0 571 639
- WO-A-98/29031
- DE-U- 29 706 108
- US-A- 4 148 540
- US-A- 4 715 825
- US-A- 5 445 535

## Beschreibung

Die vorliegende Erfindung betrifft eine Steckverbindung für eine elektrische Leitung, insbesondere für eine Anzahl von Steckzyklen ermöglichende medizinische Anwendungen.

Elektrische Steckverbindungen sind in aller Regel lösbare oder nicht lösbare elektrische Verbindungen - Kontaktierungen - eines oder mehrerer mit elektrischen Leitungen verbundener Kontakte, wobei der elektrische Kontakt durch Ineinanderstecken komplementärer Kontaktteile, wie Stecker und Buchse, bewirkt wird.

Bei lösbaren Kontaktierungen erfolgt eine Verbindung derart, dass sich ein Kontaktteil federnd an das andere legt oder mit dem anderen verklemmt wird. Hierbei gibt es sehr viele Variationen, wie z.B. Buchsen und federnde Stifte, federnde Buchsen und Stifte, Blattfedern auf Flächen, Scherklemmen, Klemmen von Drähten, Klemmen von Stiften, Schraubklemmen von Drähten oder Stiften u.s.w. Derartige Steckverbindungen sind üblicherweise für häufige Steckzyklen vorgesehen.

Im Gegensatz zu den lösbaren elektrischen Verbindungen soll bei den nicht lösbaren elektrischen Verbindungen eine Kontaktierung zwischen Leiter und Kontaktteil hergestellt werden, die in der Regel eine dauerhafte elektrische Verbindung bewirken. Zu den nicht lösbaren elektrischen Verbindungen zählen insbesondere auch Schneid-Klemm-Verbindungen, bei denen einmalig ein Kontakt mit Hilfe eines durch ein isoliertes Kabel geführtes Schneidelement hergestellt wird. Die Kontaktierung erfolgt hierbei normalerweise senkrecht zum isolierenden Mantel der elektrischen Leitungen, vgl. EP 0 571 639 A1.

Neben den im strengen Wortsinn zu verstehenden lösbaren oder nicht lösbaren elektrischen Verbindungen sind schließlich noch Mischformen bekannt, die für mehr oder minder häufige Steckzyklen ausgelegt sind, also beispielsweise solche Schneid-Klemm-Verbindungen, die für eine bestimmte Anzahl von Steckzyklen vorgesehen werden; vgl. EP-A 131 705 oder WO98/29031. Dort schneidet ein zentrischer Dorn, unmittelbar in den offenen, aus einer Vielzahl von einzelnen Drähten bestehenden elektrischen Leiter zentrisch ein.

Ein typisches Beispiel für eine lösbare elektrische Verbindung wird derzeit im medizinischen Bereich der Geburtsüberwachung für die sogenannten fetalen Kopfschwarten- oder Skalp-Elektroden zur Überwachung der fetalen Herztätigkeit verwendet. Mit der fetalen Skalp-Elektrode wird das EKG vom ungeborenen Kind mittels zweier Elektroden abgenommen. Auf der Skalp-Elektroden-Seite stehen dabei zwei offene, also abisolierte verzinnte Drähte zur Verfügung, die für einen Kontakt mit zwei federnden Backen als Gegenkontakte auf der Geräteseite vorgesehen sind. Derartige fetale Skalp-Elektro-den werden von den Anmelderin u.a. in der US-A 5,423, 314 oder der US-A 3,750,650 beschrieben.

Die fetale Skalp-Elektrode ist gewöhnlich ein Einmalprodukt und sollte daher, wie andere medizinische Einmalprodukte ebenso, möglichst preisgünstig sein. Das Verbinden von einfachen blanken Kontaktdrähten erfüllt dabei diese Kostenbedingung.

Internationale Vorschriften wie FDA (Food and Drug Administration) oder MDD (Medical Device Directive) fordern, dass bei den fetalen Skalp-Elektroden die offenen, elektrisch leitenden Drähte durch einen isolierten Steckverbinder ersetzt werden müssen, um die Patienten gegen Fehlkontaktierungen zu schützen.

Eine weitere Anforderung gerade bei fetalen Skalp-Elektroden ist weiter, dass das Steckerteil möglichst klein ist, da es bei den bekannten Ausführungsformen durch ein Innenrohr eines Einführungsinstrumentes leicht durchziehbar sein muss.

Eine derartige Steckverbindung für fetale Skalp-Elektroden, die insbesondere die genannten Sicherheitsvorschriften einhält, ist aus EP-A-484107 bekannt. Bei diesem Stecksystem werden (entsprechend der bekannten Klinikensteckerverbindung) die bei Drähte mit isolierten Buchsen verbunden, die dann auf Pins aufgesteckt werden. Nachteilig an dieser Anordnung ist jedoch, das aufgrund der geforderten

Eine weitere Anforderung gerade bei fetalen Skalp-Elek-troden ist weiter, dass das Steckerteil möglichst klein ist, da es bei den bekannten Ausführungsformen durch ein Innenrohr eines Einführungsinstrumentes leicht durchziehbar sein muss.

Eine derartige Steckverbindung für fetale Skalp-Elek-troden, die insbesondere die genannten Sicherheitsvorschriften einhält, ist aus der EP-A 484 107 bekannt. Bei diesem Stecksystem werden entsprechend der bekannten Klinkensteckerverbindung die beiden Drähte mit isolierten Buchsen verbunden, die dann auf Pins aufgesteckt werden. Nachteilig an dieser Anordnung ist jedoch, dass aufgrund der geforderten Größenlimitation des Steckerteils dieses nur sehr aufwendig und damit kostenintensiv hergestellt werden kann.

Der Erfindung liegt daher die Aufgabe zugrunde eine Kontaktierung zur Verfügung zu stellen, die es erlaubt, dass einerseits isolierte Steckverbinder verwendet werden können, z.B. zur Erfüllung der genannten Sicherheitsvorschriften, und andererseits, dass Steckerteile möglichst klein dimensionierbar sind. Auch sollte die erfindungsgemäße Kontaktierung es erlauben, möglichst preisgünstige Steckkontakte zu schaffen, die sich insbesondere für medizinische Einmalprodukte, wie z.B. fetale Skalp-Elektroden, eignen.

Diese Aufgabe wird durch die Merkmale der unabhängigen Ansprüche gelöst.

Vorteilhafte Ausführungsformen sind in den abhängigen Ansprüchen angeführt.

Die Erfindung basiert darauf, dass bei einer aus Stecker und Buchse gebildeten Klemm-Schneidverbindung das Steckerteil so ausgebildet ist, dass vor Herstellen eines elektrischen Kontaktes das Steckerteil so von einer Umformung umgeben ist, dass elektrische Leiterzumindest die zu kontaktierenden - kontaktierungsseitig elektrisch vollständig isoliert sind. Der elektrische Kontakt erfolgt dann durch Einschneiden von Kontaktierungselementen in die entsprechenden elektrischen Leiter des Steckers. Die Umformung kann mit den Steckerleitern integral verbunden oder als separate Teile ausgeführt sein, und ist vorzugsweise im Wesentlichen komplementär zu einer entsprechenden Aussparung der Buchse, in die der Stecker zur Kontaktierung einzuführen ist. Der Stecker kann - je nach Ausführungsform - in Zugrichtung der Steckerleiter, senkrecht dazu oder in jedem beliebigen Winkel in die Buchse eingeführt werden, wobei durch geeignete Gestaltung Auszugskräfte zum Trennen der Verbindung und/oder zum Herausziehen der Kabel definiert und bestimmt werden können.

Bei den bekannten Klemm-Schneidverbindungen, vgl. insbesondere die oben angeführten EP-A 131 705 oder WO98/ 29031, werden zumeist nur die durch die Kabelisolation isolierten Kabel mit entsprechenden Messern angeschnitten und das kontaktierungsseitige Ende der elektrischen Leiter, das üblicherweise nur abgeschnitten ist, kann so zu ungewünschten elektrischen Kontakten führen. Im Gegensatz hierzu erfolgt erfindungsgemäß durch die Umformung gerade auch eine elektrische Isolierung für das kontaktierungsseitige Ende der elektrischen Leiter und erlaubt so ungewünschte elektrische Kontakte zu vermeiden.

Durch im Wesentlichen komplementäre Gestaltung von Umformung und entsprechender Buchsenaussparung kann zudem der Klemm-Schneidvorgang präzisiert und auf definierte Einführposition konkretisiert werden. Geeignete Kodierungen und/oder Verriegelungsmechanismen können ferner, je nach Anwendung, verwendet werden.

Die erfindungsgemäße Lösung eignet sich insbesondere dazu, die für fetale Skalp-Elektroden notwendigen Sicherheitskontaktierungen zu ermöglichen. Allerdings ist die erfindungsgemäße Lösung nicht auf medizinische Anwendung im Allgemeinen oder fetale Skalp-Elektroden im Speziellen beschränkt, sondern eignet sich für alle lösbaren Steckverbindungen, bei denen für die Steckerseite nur eine geringere Steckzyklenzahl notwendig ist. Dies trifft insbesondere für Einmalprodukte oder sonstige Anwendungen zu, bei denen eine lösbare Kontaktierung geschaffen werden soll, diese jedoch regelmäßig nur ein oder wenige Male geschlossen oder geöffnet wird.

Bei Verwendung der erfindungsgemäßen Lösung, insbesondere bei fetalen Skalp-Elektroden, wird sichergestellt, dass die Sicherheitsvorschriften bei geringen Kosten eingehalten werden können. Außerdem wird der elektrische Verbindungsvorgang für den Anwender wesentlich vereinfacht. Da der Benutzer normalerweise Schutzhandschuhe trägt und diese durch das vaginale Einführen der fetalen Skalp-Elektrode feucht sind, ist das Bedienen der herkömmlich üblichen Federbacken und das Einlegen der abisolierten Drähte nicht einfach. Dies wird durch eine erfindungsgemäße Steckverbindung wesentlich verbessert, da der Anwender auch mit einer Hand den Stecker sicher bedienen kann.

Unabhängig von dem jeweiligen Einsatzgebiet erlaubt es die erfindungsgemäße Lösung Steckkontakte, die für geringere Steckzyklenzahlen benötigt werden, zu geringen Kosten zu schaffen. Insbesondere erlaubt es die Erfindung auch nur einen Teil der Steckerverbindung, vorzugsweise das isolierte Steckerteil, sehr preisgünstig herstellen zu können. Das zweite Teil der Steckverbindung ist dabei zu Kosten herzustellen, die den momentan eingesetzten Lösungen entsprechen. So entspricht beispielsweise der Aufwand des Verzinnens der abisolierten Drähte einem Umspritzen der Kabel mit einem erfindungsgemäßen Stecker. Das bedeutet, dass der Vorteil des erfindungsgemäßen Lösungsprinzips insbesondere in den Kosten für solche Steckverbindungen liegen kann, bei denen beispielsweise der Stecker preisgünstig sein sollte und nur für eine geringe Steckzyklenzahl benötigt wird. Somit eignet sich die Erfindung insbesondere für Einmalartikel, wie sie gerade im medizinischen Bereich aus hygienischen Gründen häufig anzutreffen sind.

Eine bevorzugte Ausführungsform einer erfindungsgemäßen Steckverbindung weist auf der einen Seite, der Kabelseite, ein auf ein, vorzugsweise nicht abisoliertes, Kabel sitzendes Steckerteil, dass vorzugsweise mit Kunststoff auf das Kabel überspritzt ist, und auf der anderen zu kontaktierenden Seite ein Buchsenteil zur Aufnahme des Steckerteils auf. Insbesondere bei Verwendung des gleichen Kunststoffmaterials für das Steckerteil wie für die Kabelisolation kann eine gute Verbindung zwischen Steckerteil und Kabelisolation erreicht werden. Das Steckerteil auf der Kabelseite ist dabei so geformt, dass es vorzugsweise verdrehgesichert in die Öffnung des Buchsenteils eingeschoben werden kann. Während des Einschiebevorgangs schneidet eine in einen offenen Raum des Buchsenteils hineinragende Kontaktschneide durch die Isolation in den Kabelleiter und erzeugt damit eine elektrische Verbindung zwischen einem Kontaktteil des Buchsenteils und dem Kabelleiter. Entsprechend können bei einer Vielzahl von Kabelleitern eine Vielzahl von Kontaktschneiden vorgesehen werden, die jeweils eine elektrische Verbindung zwischen einem entsprechenden Kontaktteil des Buchsenteils und dem jeweiligen Kabelleiter herstellen.

In einer bevorzugten Ausführungsform ist das Buchsenteil als entnehmbares bzw. auswechselbares Teil vorgesehen, um so ein schnelles Auswechseln der Buchse, z.B. im Falle einer nachlassenden Schneidintensität oder für ein Reinigen oder Sterilisieren des Buchsenteils, zu gewährleisten. Je nach Anwendung kann das Buchsenteil auch als Einmalartikel ausgeführt sein.

In einer anderen Ausführungsform kann das Buchsenteil steckbar mit einem Verbindungselement, wie z.B. mit einem Messgerät oder einer Beinplatte, bei der fetalen Skalp-Elektrode, verbunden werden, um so ein schnelles und einfaches Auswechseln zu gewährleisten. Dabei kann durch entsprechende Auslegung und/oder Dimensionierung dieser Steckverbindung eine evtl. vorgegebene Auszugkraft erreicht werden, bei der sich das Buchsenteil unter Zugeinwirkung aus der Verbindung mit dem Verbindungselement löst. Dies kann insbesondere bei medizinischen Anwendungen wie der fetalen Skalp-Elektrode erforderlich oder erwünscht sein.

Der Stecker wird vorzugsweise derart ausgeführt, dass er auch nach einer Anzahl von erfolgten Schneid-Klemm-Verbindungen noch elektrisch isolierend wirkt, wenngleich auch gegebenenfalls in eingeschränktem Umfang. Dies lässt sich beispielsweise durch geeignete Materialauswahl und/oder Dimensionierung der Kabelisolation und/oder der Umformung erreichen, so dass z.B. Kabelisolation und/oder Umformung nach Lösen der Schneid-Klemm-Verbindung zu einem bestimmten Grad in ihre Ausgangsposition vor Herstellung der Schneid-Klemm-Verbindung zurückgehen. Entsprechend lässt sich durch Vorsehen geeigneter Luftstrecken die Gefahr von ungewollten elektrischen Kontakten nach Lösen der Schneid-Klemm-Verbindung begegnen.

Die Erfindung wird im Folgenden weiter unter Heranziehung der Zeichnungen erläutert, wobei sich gleiche Bezugszeichen auf funktional gleiche oder ähnliche Merkmale beziehen.
Es zeigen:
- Fig. 1: eine bevorzugte Ausführungsform einer erfindungsgemäßen elektrischen Steckverbindung 10,
- Fig. 2: in größerer Detailansicht und in mehreren Ansichten das in Fig. 1 gezeigte Steckerteil 20,
- Fig. 3A und 3B: in größerer Detailansicht die in Fig. 1 dargestellte Buchse 30,
- Fig. 4A und 4B: alternative Ausführungsformen von Schneiden 50,
- Fig. 5: eine geeignete Ausformung einer Schneide 50,
- Fig. 6: eine Ausführungsform des Buchsenteils 30, und
- Fig. 7A, 7B und 7C: eine Ausführungsform die keinen Teil der Erfindung bildet

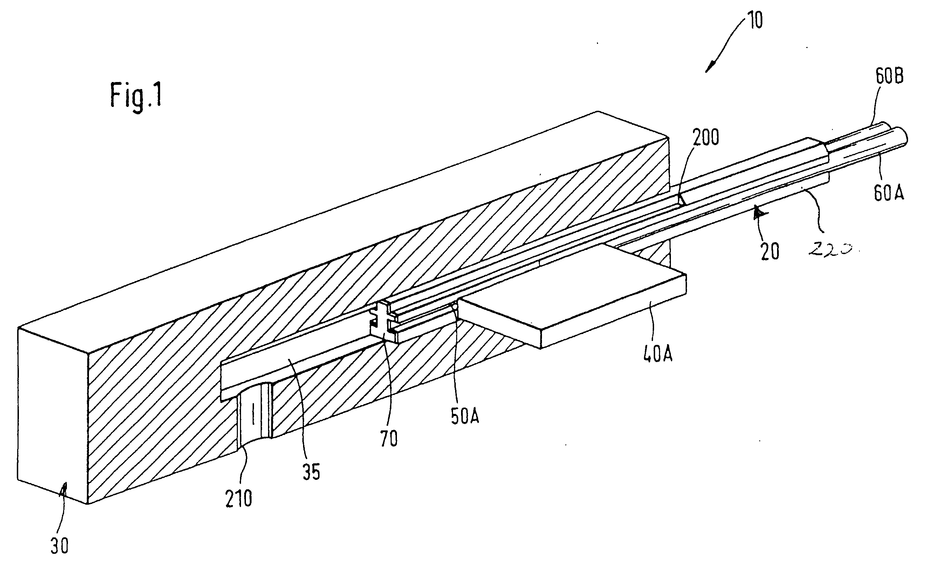

Fig. 1 zeigt eine bevorzugte Ausführungsform einer erfindungsgemäßen elektrischen Steckverbindung 10. Ein Steckerteil 20 - im Folgenden auch nur Stecker genannt - ist dabei teilweise in ein aufgeschnitten dargestelltes Buchsenteil 30 - im Folgenden auch nur Buchse genannt - eingeführt. Die Formen von Stecker 20 und Buchse 30 sind aufeinander angepasst, wobei die Begriffe Stecker 20 und Buchse 30 hier so verwendet werden sollen, dass der Stecker 20 in einen Buchsenkanal 35 der Buchsen 30 einführbar oder einsteckbar ist. Ein mit der Buchse 30 verbundenes Kontaktteil 40A trägt an seiner dem Stecker 20 zugewandten Seite eine - in Fig. 1 nur teilweise sichtbare - Kontaktschneide 50A. Die Kontaktschneide 50A soll im kontaktierten Zustand einen elektrischen Kontakt zwischen dem, vorzugsweise nach außen zu führenden, Kontakt 40A der Buchse 30 und zumindest einem entsprechenden elektrischen Leiter des Steckers 20 herstellen.

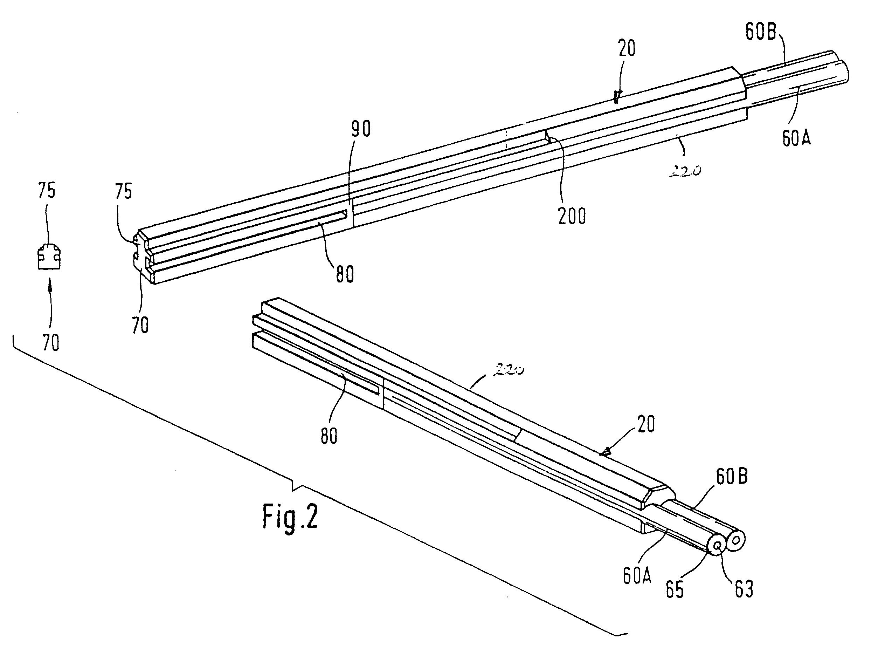

In dem in Fig. 1 gezeigtem Ausführungsbeispiel weist der Stecker 20 eine Umformung 220 und zwei Kabel oder Drähte 60A und 60B auf, die jeweils ummantelte und damit isolierte elektrische Leiter enthalten; vgl. 63 in Fig. 2. Die Schneide 50A des Kontaktes 40A soll einen elektrischen Kontakt zu dem Leiter 60A herstellen und ein weiterer Kontakt 40B mi einer entsprechenden Schneide 50B in der Fig. 1 gewählten Darstellungsweise - jeweils nicht sichtbar - ist zur Kontaktierung des Leiters 60B vorgesehen.

Die Umformung 220 des Steckers 20 wird vorzugsweise durch Überspritzen, beispielsweise mit einem Kunststoff, der isolierten Kabel 60 hergestellt, was eine kostengünstige Fertigung des Steckers 20 erlaubt. Anstelle der in Fig. 1 gezeigten zwei isolierten Drähte 60A und 60B können je nach Anwendung ein Draht 60 oder mehrere Drähte 60 verwendet werden.

Fig. 2 zeigt in größerem Detail und in mehreren Ansichten den in Fig. 2 gezeigte Stecker 20. Die äußere Ausformung des Steckers 20 ist im wesentlichen komplementär zu der äußeren Ausformung der Buchse 30, um so ein Ineinanderstecken von Stecker 20 und Buchse 30 zu gewährleisten.

In der in den Fig. 1 und 2 gezeigten Ausführungsform weist der Stecker 20 vorzugsweise weiter eine geeignete Außenformung auf, um so dem Stecker 20 in seiner Positionierung gegenüber der Buchse 30 zu sichern und ein ungewünschtes Verdrehen auszuschließen. Auf diese Weise kann bei mehreren Kabeln 60 beispielsweise ein polaritätsrichtiges Kontaktieren der einzelnen Kabel oder bzw. dass die einzelnen Kabel 60 den jeweils entsprechenden Kontakten 40 zugeordnet werden, sichergestellt werden. In dem in den Fig. 1 und 2 gezeigten Beispiel weist der Stecker 20 an seiner der Buchse 30 zugewandten Stirnseite 70 in etwa die Form zweier spiegelbildlich aneinander gerückter und auf dem Kopf stehender F's oder, anders ausgedrückt, eines auf dem Kopf stehenden T's mit einer Mittelstrebe parallel zu der Kopfhorizontalen auf. Ein sich somit ergebendes, zu einer entsprechenden Mittelhorizontalen H in Einführungsrichtung nicht achsensymmetrisches und in Fig. 2 oberhalb der oberen Horizontalen befindliches Kopfteil oder Steg 75 gewährleistet ein orientierungsrichtiges Einfügen des Steckers 20 in die Buchse 30.

Durch geeignete Formgebung des Steckers 20, insbesondere entsprechend der Stirnseite 70, lassen sich ferner auch Kodierungen unterschiedlicher Steckertypen erreichen, um so beispielsweise ein ungewünschtes Anschließen an eine nicht geeignete Buchse 30 zu verhindern.

Für den Fall, dass eine Verdrehsicherung des Steckers 20 nicht erforderlich ist, kann der Stecker 20 und insbesondere die Stirnseite 70 auch entsprechend achsen- oder punktsymmetrisch aufgebaut sein, so dass ein Kontaktieren des Steckers 20 mit der Buchse 30 in mehreren Drehwinkeln, beispielsweise jeweils 180°, ermöglicht wird. In diesem Falle wäre beispielsweise eine H-Form, z.B. auf der Seite liegend wie in Fig. 2 gezeigt, oder dergleichen geeignet.

Die geeignet ausgeführte Formgebung des Steckers 20 vorzugsweise durch Ummantelung oder Überspritzen der Kabel 60, umfasst steckerseitig die isolierten Kabel 60, legt deren Position fest und gibt ihren Halt. Auf der Seite des Steckers 20, auf der die Kontaktschneide 50 in den jeweiligen Draht 60 eingreift, befindet sich vorzugsweise eine Aussparung oder Ausnehmung 80 in der Umformung 220, die verhindern soll, dass das oder die Kontaktschneide 50 unnötig vor der eigentlichen Kontaktierung belastet werden.

Um eine hinreichende Isolation der Kabelenden der Kabel 60 in Steckerrichtung sicherzustellen, werden diese Kabelenden vorzugsweise nicht bis zu der Stirnseite 70, sondern nur bis zu einem vorgebbaren Abstand dazu ausgeführt. Um die Isolation weiter zu erhöhen, ist in einer bevorzugten Ausführungsform, vorzugsweise zwischen dem Ende des jeweiligen Kabels 60 und dieser Ausnehmung 80 der Umformung, eine dünne Schicht 90, vorzugsweise aus einem Kunststoff, vorgesehen.

Wie aus dem unteren Teil der Fig. 2 ersichtlich, sind die Kabel 60 vorzugsweise jeweils aus einem elektrischen Leiter 63 und einer den Leiter 63 umgebenden Isolierung 65 aufgebaut. Der Leiter 63 kann dabei ein Einzelleiter sein oder aus einer Vielzahl von einzelnen Leiterfasern oder Litzen bestehen. Allerdings ist die Erfindung nicht auf die Kontaktierung derartiger in Fig. 2 gezeigter Kabel beschränkt, sondern es können beliebige andere Kabel verwendet werden. Insbesondere muss sich die Umformung 220 nicht an die Isolierung 65 anschließen, sondern kann auch direkt an dem Leiter 63 anliegen, so dass beispielsweise auch solche Kabel verwendet werden können, bei denen steckerseitig die Isolierung 65 entfernt wurde.

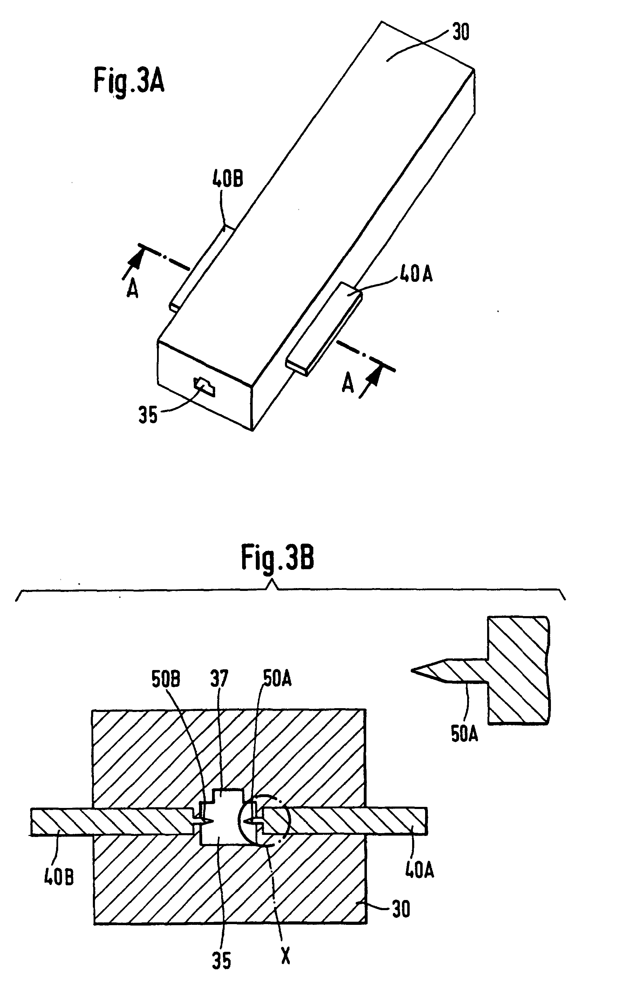

Die Fig. 3A und 3B zeigen in größerem Detail die in Fig. 1 dargestellte Buchse 30, wobei Fig. 3A eine perspektivische Darstellung der Buchse 30 und Fig. 3B eine Schnittbilddarstellung an der Stelle A-A und in Sichtrichtung der Pfeile A zeigt. Die Kontakte 40A und 40B stehen sich an entgegengesetzten Seiten des Buchsenkanals 35 gegenüber, wobei jeweils die Schneiden 50A und 50B in den Buchsenkanal 35 hineinreichen.

Die Formgebung des Buchsenkanals 35 korrespondiert im Wesentlichen mit der Formgebung des Steckers 20, um ein leichtes Ineinanderfügen zu gewährleisten. Gleichzeitig muss die Formgebung des Buchsenkanals 35 auf eine gegebenenfalls ausgeführte Verdrehsicherung und/oder Kodierung des Steckers 20 ausgerichtet sein und weist dementsprechende Verdrehsicherungs- und/oder Dekodierungselemente auf. In dem Ausführungsbeispiel der Fig. 3A und 3B weist der Buchsenkanals 35 entsprechend der Formgebung des in Fig. 2 gezeigten Steckers 20 eine zu dem Steg 75 der Umformung 220 komplementäre Ausnehmung 37 auf.

In den Fig. 3A und 3B ist das den Schneiden 50 entgegengesetzte Ende der Kontakte 40 zur externen Kontaktierung aus dem Gehäuse der Buchse 30 herausgeführt. Allerdings sind auch beliebige andere Herausführungen oder dergleichen der Kontakte 40 aus der Buchse 30 entsprechend der jeweiligen Anwendung möglich. So können beispielsweise die Kontakte 40 in der Buchse 30 in Richtung der Kabelachse innerhalb der Buchse 30 durchgeführt und an einer der Steckereinführungsöffnung des Buchsenkanals 35 entgegengesetzten Seite der Buchse 30 herausgeführt werden.

Durch Einschieben, vgl. Fig. 1, des Steckers 20, vgl. Fig. 2, in das Buchsenteil 30, vgl. Fig. 3B, wird die Kontaktierung erzeugt. Die Kontaktschneiden 50 werden vorzugsweise als Messer 50 ausgeführt und sind derart angeordnet, dass sie während des Einführungsvorgangs zunächst in der Aussparung oder Schlitz 80, vgl. Fig. 2, der Umformung 220 des Steckers 20 gleiten, um dann aus Sicherheitsgründen, um Berührungsschutz zu gewährleisten, nach einer gewissen Tiefe in die isolierten Kabel 60 einzuschneiden und elektrischen Kontakt zu erzeugen.

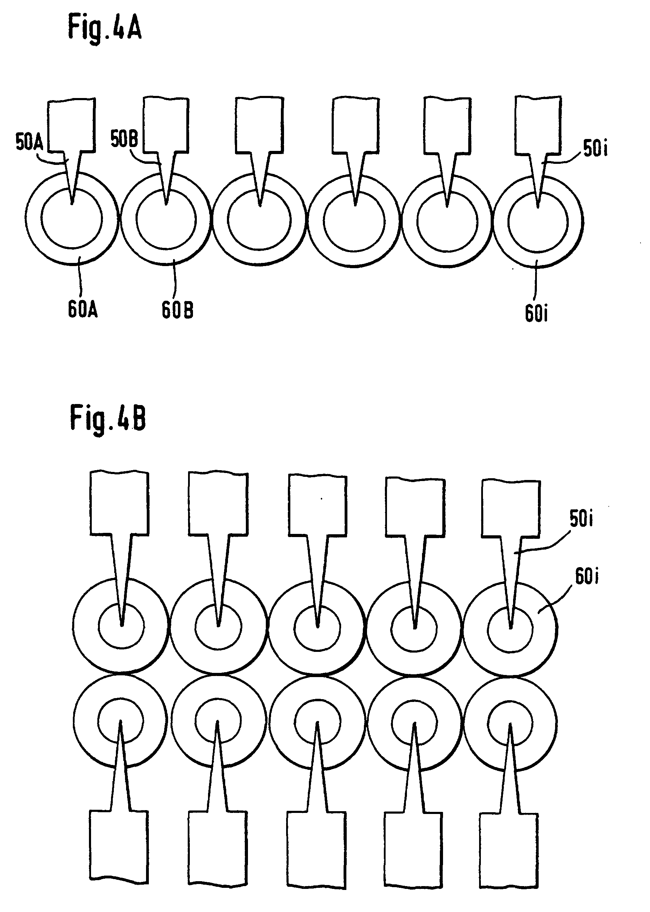

Die Fig. 4A und 4B zeigen alternative Ausführungsformen von Kontaktschneiden 50 für die Kontaktierung mit Kabeln 60 mit einer Vielzahl von Einzelkabeln 60i - mit i = A, B,... - in Fig. 4A sind die Kabel in einer Reihe parallel zueinander angeordnet. Entsprechend weist die Buchse 30 eine Vielzahl von Kontaktschneiden 50i aneinandergereiht in einer Reihe auf. In Fig. 4B hingegen weist der Stecker 20 zwei zueinander parallele Reihen von Kabeln 60i auf, und entsprechend weist die Buchse 30 jeweils eine Reihe von Kontaktschneiden 50i oberhalb und unterhalb der Kabelreihen 60i auf.

In einer bevorzugten Ausführungsform wird für die Isolation der Kabel 60i das gleiche Material, vorzugsweise ein Kunststoff, wie für die Umformung 220 verwendet, wobei bei Verwendung eines Kunststoffs vorzugsweise die Umformung 220 mit diesem Kunststoff über die Kabel 60i überspritzt wird. Durch die entsprechende Auswahl der Materialien für die Ummantelung/Isolation der Kabel 60i und der Umformung 220 und vorzugsweise dadurch, dass gleiche Materialien verwendet werden, kann eine enge Verbindung dieser Materialien gewährleistet werden.

In einer bevorzugten Ausführungsform sind die Kontaktschneiden oder -Messer 50i aus einem Material hergestellt, das sehr hart und rostfrei ist und einen guten Kontakt gewährleistet. Ferner sind die Kontaktmesser 50i derart geformt, dass sie so lange wie möglich scharf bleiben, um eine genügend große Anzahl von Steckzyklen zu gewährleisten.

Durch das Vorsehen der Aussparungen 80 und die damit verbundene exakte, genügend lange Führung des Steckers 20 im Buchsenkanal 35 des Buchsenteils 30 wird sichergestellt, dass auf die Schneiden 50i der Kontakte 40i keine Querkräfte kommen und somit ein Bruch der Schneiden 50i vermieden werden kann. Bei der Schneide 50i selbst muss unterschieden werden zwischen dem schneidenden Teil und dem Teil, der den Kontakt herbeiführt. Der schneidende Teil sollte möglichst lang sein, um immer einen scharfen Anteil zu haben, während der Teil, der den Kontakt herstellt, möglichst schräg, z.B. in einem Winkel kleiner als 45°, zu eventuell im Kabel 60i enthaltenen Litzen stehen sollte, um zu vermeiden, dass alle Fasern oder Litzen zur gleichen Zeit aufgespalten werden müssen.

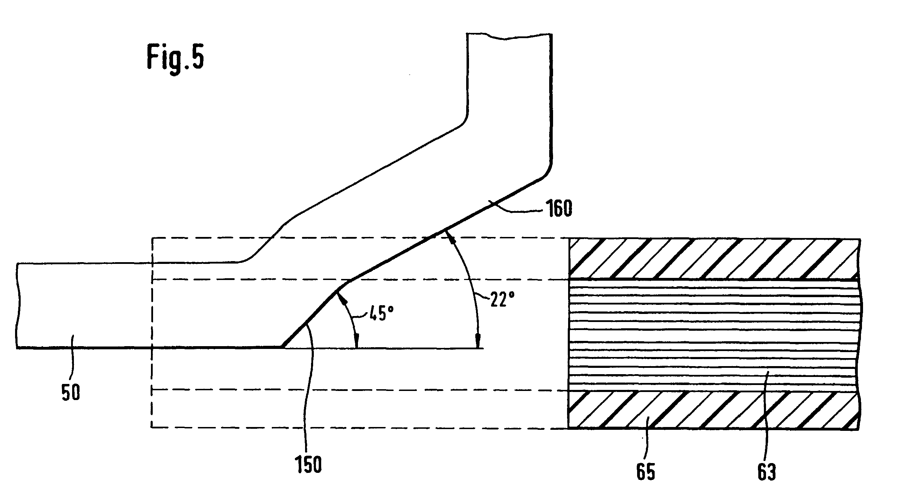

Fig. 5 zeigt eine geeignete Ausformung einer Kontaktschneide 50. Die Schneide 50 weist einen ersten, vorzugsweise um etwa 40 bis 50°, vorzugsweise 45° abgewinkelten Bereich für die Kontaktbildung mit dem Leiter 63 des Kabels 60 auf. An den ersten Bereich 150 schließt sich ein zweiter Bereich 160 an, der vorzugsweise um etwa 10 bis 30°, vorzugsweise 22° abgewinkelt ist und dazu dient, die Isolation 65 und einen kleinen Teil der Leiter 63 anzuschneiden. In Versuchen konnte gezeigt werden, dass mit einer derartigen Ausformung der Schneide 600 Steckzyklen ohne weiteres möglich sind.

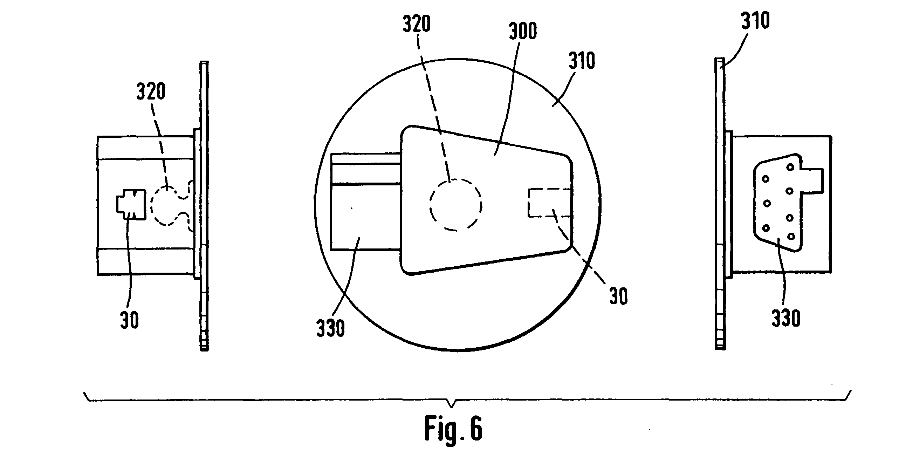

Fig. 6 zeigt in Ansichten von oben und den Seiten eine bevorzugte Ausführungsform der Buchse 30, die erlaubt, dass der Stecker 20 sich bei Zug entgegengesetzt der Buchse 30 aus diesem löst. Zudem ermöglicht diese Lösung, dass die Buchse 30 leicht ausgewechselt werden kann, z.B. im Falle nachlassender Schneidintensität oder zum Reinigen oder Sterilisieren der Buchse 30. Ein Kunststoffkörper 300, der auf der einen Seite die erfindungsgemäße Buchse 30 trägt, wird auf einen Trägerkörper 310, wie z.B. eine Standard-EKG-Klebeelektrode, mit Hilfe einer Druckknopfverbindung 320 aufgeschnappt. Im Falle der Standard-EKG-Klebeelektrode 320 bildet diese zusammen mit dem Kunststoffkörper 300 beispielsweise eine passive Beinplatte zur Befestigung am Bein der Patientin. Vorzugsweise trägt der Kunststoffkörper 300 auf der anderen Seite eine weitere Kontaktverbindung 330, wie z.B. eine Buchse gemäß US-A 5,615,674.

Bei Verbindung von Stecker 20 und Buchse 30 und bei Zugbeanspruchung z.B. am Kabel 60, nicht gezeigt in Fig. 6, dreht sich der Kunststoffkörper 300 um die Druckknopfverbindung 320 in die Zugrichtung. Dadurch greift der Zug immer in der Auszugsrichtung des Steckers 20 an und ermöglicht so ein leichtes Öffnen der Steckverbindung.

Der Stecker 20 wird vorzugsweise durch die mit dem Schneidvorgang bewirkten Reibungskräfte in der Buchse 30 gehalten. Allerdings kann neben oder an Stelle dieser Klemmverbindung auch eine andere Halterung, z.B. durch ein Verriegeln und/oder Festsetzen beispielsweise durch Zapfen oder Pins, vorgesehen werden.

Ein bevorzugtes Ausführungsbeispiel eines erfindungsgemäßen Kontaktierungssystems soll nun exemplarisch für die Kontaktierung einer fetalen Skalp-Elektrode dargestellt werden. Dabei soll insbesondere auf die Fig. 1 bis 3 und 5 Bezug genommen werden, wobei die genannten Fig. nicht auf die Verwendung für die fetale Skalp-Elektrode beschränkt sind.

Der Stecker 20 als das preisgünstigere Teil zeigt rechts und links in der Darstellung in Fig. 2 die Ausnehmungen 80 für die Kontaktschneiden 50, die auf der Buchsenseite in den Fig. 3 dargestellt sind. In dem Bereich der Ausnehmungen 80 sind die Schneiden 50 frei und nicht im Eingriff mit dem Stecker 20. Beim weiteren Einführungsvorgang des Steckers 20 in die Buchse 30 über den Bereich der Ausnehmungen 80 hinausgehend wird dann die vorzugsweise sehr kurze Isolationsschicht 90 durchschnitten, um dann die mit der Isolierung 65 versehenen Leiter 63 der Kabel 60 aufzuschneiden und bei weiterem Einschieben des Steckerteils 20 den Kontakt zu den Kupferlitzen des Leiters 63 herzustellen.

Als Verdrehsicherung und zur Verbesserung der Führung zwischen Buchse 30 und Stecker 20 ist am oberen Bereich des Steckers 20 der Steg 75 und am oberen Bereich des Buchsenkanals 35 die zu dem Steg 75 komplementäre Ausnehmung 37 vorgesehen, die sicherstellt, dass der Stecker 20 nur in einer Ausrichtungsweise in die Buchse 30 eingeführt werden kann.

Ein in den Fig. 1 und 2 gezeigter Anschlag 200 des Steckers 20 ist dafür vorgesehen den Einführvorgang zu stoppen. Dabei stößt der Anschlag 200 des Steckers 20 an die dem Stecker zugewandte Frontfläche der Buchse 30 an. Dieser Stop 20 ist insbesondere bei der fetalen Anwendungsweise sinnvoll, da bei einer Verschmutzung des Buchsenkanals 35 dieser Schmutz in den hinteren Bereich des Buchsenkanals 35 geschoben und dort durch eine in Fig. 1 gezeigte Öffnung 210 nach außen verdrängt werden kann. Dies wäre bei einem Anschlag an der Endfläche des Buchsenkanals 35 nicht gesichert.

Zur Herstellung der Umformung 220 werden die in Fig. 1 gezeigten Kabel 60A und 60B in ein entsprechendes Werkzeug eingelegt und rechts und links in Position gehalten. Dann erfolgt ein Kunststoffspritzvorgang, der eine Verbindung zwischen Kunststoffkörper und der Isolierung 65 der Kabel 60 herbeiführt. Dadurch wird der Stecker 20 mechanisch stabil und kann gut in die Buchse 30 eingeschoben werden. Eine Kabeltülle oder Knickschutz kann vorgesehen werden, ist jedoch für die Anwendung der fetalen Skalp-Elektrode nicht erforderlich, da der hier gewünschte Stecker 20 vorzugsweise sehr klein und duktil ist.

Die für die Kontaktierung des Steckers 20 mit der fetalen Skalp-Elektrode vorgesehene Buchse 30 ist entsprechend der Fig. 3A und 3B aufgebaut, kann jedoch auch andere Ausführungsformen aufweisen. Die Schneiden 50, die mit den Kontakten 40 fest verbunden sind, werden vorzugsweise in der richtigen Position fest in die Buchse 30 eingespritzt. Die Schneiden 50 selbst haben vorzugsweise die in Fig. 5 beschriebene Form.

Die Buchse 30 wiederum wird vorzugsweise entsprechend Fig. 6 zusammen mit einer Standard-Fetal-Buchse 330 gemäß US-A 5,615,674 in einem Kunststoffkörper 300 auf einer Standard-EKG-Klebeelektrode 310 mit Hilfe einer Druckknopfverbindung 320 aufgeschnappt. Die Standard-EKG-Klebeelektrode 320 bildet zusammen mit dem Kunststoffkörper 300 eine passive Beinplatte zur Befestigung am Bein der Patientin.

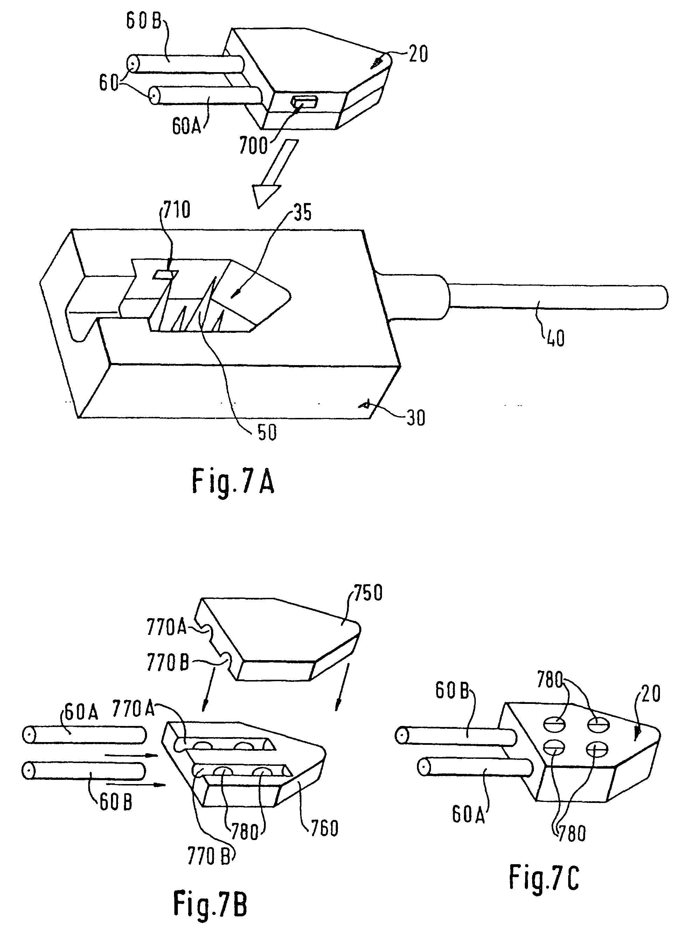

Die Fig. 7A bis 7C zeigen eine Ausführungsform die keinen Teil der Erfindung bildet. Während bei der in Fig. 1 dargestellten Klemm-Steckverbindung der Kontaktierungsvorgang im Wesentlichen in Richtung der Kabelachse durchgeführt wird, erfolgt bei der in den Fig. 7 dargestellten Klemm-Steckverbindung der Kontaktierungsvorgang im Wesentlichen senkrecht zur Kabelachse.

In der 3-dimensionalen Darstellung der Fig. 7A wird das Steckerteil 20 in Pfeilrichtung in den Buchsenkanal 35 des Buchsenteils 30 eingeführt. Schneiden 50 stellen im kontaktierten Zustand einen elektrischen Kontakt zwischen nach außen über ein Kabel angeführte Kontakte 40 der Buchse 30 und den entsprechenden elektrischen Leitern 60 des Steckers 20 her. In dem in den Fig. 7 gezeigten Ausführungsbeispiel weisen der Stecker 20 und die Buchse 30 je zwei isolierte Kabel auf.

Durch die im Wesentlichen komplementäre Formgebung des Steckers 20 und des Buchsenkanals 35 werden die elektrischen Kontakte von Stecker 20 und Buchse 30 bereits fest aufeinander bezogen, so dass bei mehreren zu kontaktierenden Kabeln 60 nur zwei Möglichkeiten der Kombination, nämlich entsprechend der in den Fig. 1 und 7A gezeigten Steckrichtung oder mit 180° um die Kabelachse gedrehtem Stecker 20 zugelassen werden. Entsprechend dem Vorbeschriebenen, können diese Kombinationsmöglichkeiten durch entsprechende Kodierung oder andere geeignete Maßnahmen auf eine zugelassene eingeschränkt werden.

In Fig. 7A erfolgt eine derartige Einschränkung der Kombinationsmöglichkeiten bevorzugt durch eine Nase oder Vorsprung 700 in der Umformung 220, wobei der Vorsprung 700 bei seitenrichtiger Einführungsweise - in Fig. 7A müsste der Stecker 20 hierfür um 180° gedreht werden - mit einer entsprechenden Einkerbung oder Vertiefung - in Fig. 7A nicht gezeigt - in dem Buchsenkanal 35 zusammenwirkt. Anstelle einer derartigen Kodierung, oder in Kombination dazu, kann der Vorsprung 700 auch federnd ausgeführt werden, so dass dieser mit einer entsprechenden Vertiefung 710 in dem Buchsenkanal 35 zusammenwirken kann und bei richtiger Einführrichtung ab einer bestimmten, vorgebbaren Einführtiefe in die Vertiefung 710 einrastet, beispielsweise im Sinne einer Schnappverbindung. Erfolgt keine Einführkodierung, kann der Vorsprung 700 so elastisch ausgelegt werden, dass beide Einführrichtungen zugelassen werden. In diesem Falle erfolgt in der ersten Einführungsrichtung bei entsprechender Zusammenführung von Vorsprung 700 und Vertiefung 710 eine evtl. sicherheitserhöhende Verrasterung, während die zweite Einführungsrichtung ohne Zusammenführung von Vorsprung 700 und Vertiefung 710 ebenfalls zugelassen wird, allerdings ohne Verrasterung.

Das Zusammenfügen von Stecker 20 und Buchse 30 in Fig. 7A erfolgt in Pfeilrichtung. Zur Herstellung der elektrischen Kontakte wird dabei der Stecker 20 gegen die im Buchsenkanal 35 sich befindenden Schneiden oder Messer 50 gedrückt. In dem in Fig. 7A dargestellten Ausführungsbeispiel sind dabei für jedes Kabel 60 jeweils zwei Messer 50 vorgesehen, die an unterschiedlichen Positionen in Kabelrichtung angreifen. Dabei kann das Zusammenführen einfach durch Drücken von Hand oder durch eine geeignete Vorrichtung, wie einen Hebelmechanismus oder dergleichen, erfolgen bzw. unterstützt werden. Ferner kann nach erfolgter Verbindung von Stecker 20 und Buchse 30 die Verbindung durch eine geeignete Abdeckung oder einen Verschluss gegen Eindringen von Verunreinigungen und/oder ungewünschtes Öffnen gesichert werden. Der/dem Abdeckung/Verschluss kann zudem eine Löse-/Auswurfsfunktionalität zubemessen werden, so dass eine Trennung von Stecker 20 und Buchse 30 mechanisch unterstützt wird.

Fig. 7B zeigt in Explosionsdarstellung eine bevorzugte Ausführungsform des in Fig. 7A gezeigten Steckers 20. Der Stecker 20 ist hier aus einem Oberteil 750 und einem Unterteil 760 zusammengesetzt. Die zwei isolierten Kabel 60A und 60B - entsprechend könnten es auch nur ein oder mehr als zwei Kabel 60 sein - werden zum Zusammenbau des Steckers 20 in entsprechend ausgeführte Kabelkanäle 770A und 770B entweder des Oberteils 750 oder des Unterteils 760 eingelegt, das dann mit dem entsprechenden Gegenstück des Steckers 20 - ebenfalls mit entsprechenden Kabelkanälen 770A und 770B versehen - zusammengesteckt wird, wobei das Oberteil 750 und das Unterteil 760 die Kabel 60A und 60B fixieren. Durch entsprechende Dimensionierung der Passung zwischen Oberteil 750 und Unterteil 760, insbesondere der Durchmesser der Kabelkanäle 770A und 770B, kann die zum Herausziehen der Kabel 60 entgegen der Pfeilrichtung erforderliche Auszugskraft eingestellt werden. Ebenso kann über entsprechende Formgebung und Gestaltung von Oberteil 750 und Unterteil 760 eine unlösbare oder eine mehr oder minder leicht lösbare Verbindung zwischen Oberteil 750 und Unterteil 760 erreicht werden, so dass der Stecker 20 nach Zusammenbau als Einmalteil ausgelegt ist oder gegebenenfalls wiederverwendbar ist.

Fig. 7C zeigt eine besondere Ausführungsform des in Fig. 7B gezeigten Unterteils 760, allerdings um 180° gedreht und bereits mit den Kabeln 60 und dem Oberteil 750 verbunden. Das Unterteil 760 weist dabei für jedes Kabel 60 zumindest eine Aussparung 780 im Bereich des Kabelkanals 770 auf, so dass die Kontaktschneiden 50 unmittelbar in die Kabelisolierung eindringen können und nicht erst die Umformung 220 durchschneiden müssen. Dadurch werden die Messer 50 geschont. Zudem kann hierdurch auch der Stecker 20 dickwandiger und/oder aus härterem Material ausgeprägt werden.

Wie aus Fig. 7A zu entnehmen ist, können Buchse 30 und Stecker 20 jeweils so ausgebildet werden, dass sie an den Kabelenden der Kabel 40 und 60 sich befinden.

### BEZUGSZEICHENLISTE

- 10: Steckverbindung
- 20: Stecker
- 30: Buchse
- 35: Buchsenkanal
- 37: Ausnehmung
- 40: Kontakt
- 40A: Kontaktteil
- 40B: Kontakte
- 40i: Kontakte
- 50: Kontaktschneide
- 50A: Kontaktschneide
- 50B: Schneide
- 50i: Kontaktschneiden
- 60: Kabel
- 60A: Kabel oder Draht
- 60B: Kabel oder Draht
- 60i: Kabel
- 63: elektrischer Leiter
- 65: Isolierung
- 70: Stirnseite
- 75: Steg
- 80: Ausnehmung
- 90: Schicht
- 150: Bereich
- 160: Bereich
- 200: Anschlag
- 210: Öffnung
- 220: Umformung
- 300: Kunststoffkörper
- 310: Trägerkörper
- 320: Druckknopfverbindung
- 330: Kontaktverbindung
- 600: Schneide
- 700: Nase oder Vorsprung
- 710: Vertiefung
- 750: Oberteil
- 760: Unterteil
- 770: Kabelkanal
- 770A: Kabelkanal
- 770B: Kabelkanal
- 780: Aussparung

## Patentansprüche

1. Steckverbindung (10) für eine elektrische Leitung (Kabel 60), insbesondere für eine Anzahl von Steckzyklen ermöglichende medizinische Anwendungen, mit einem Steckerteil und einem Buchsenteil (30) mit an entgegengesetzten Seiten gegenüberstehenden Kontaktierungsteilen (40), die mittels Kontaktschneiden (50) in einen die Aufnahme des verdrehgesichtert einschiebbaren, der Kontaktierung dienenden Steckerteils (20) ermöglichenden, komplementär zum Steckerteil ausgebildeten Kanal (35) des Buchsenteiles (30) ragen, welches Steckerteil (20) als eine elektrisch isolierende Umformung ausgebildet ist, die die jeweils von einer Isolierung ummantelten Leiter (63) der elektrischen Leitung (60) kontaktierungsseitig positionierend umgibt und einen Anschlag (200) aufweist, dies alles in derartiger Anordnung, dass bei dem durch den Anschlag (200) begrenzten Einschieben des Steckerteils (20) in den Kanal (35) des Buchsenteils (30) die in den Kanal (35) ragenden Kontaktschneiden (50) der Kontaktierungsteile (40) in die jeweils zugewandte Seite der Isolierung (65) des Kabels (60) zwecks Kontaktierung von Leiter (63) des Kabels (60) und zugeordneten Kontaktierungsteil (40) des Buchsenteiles (30) einschneiden.

2. Steckverbindung nach Anspruch 1,
**dadurch gekennzeichnet,**
**dass** das Steckerteil (20) zwecks positionsgenauem Ineinanderführens von Buchsenteil (30) und Steckerteil (20) einen als Verdrehsicherung (75) dienenden Querschnitt aufweist, der zumindest zu einer Mittelachse nicht achsensymmetrisch ausgebildet ist.

3. Steckverbindung nach den Ansprüchen 1 und 2,
**dadurch gekennzeichnet,**
**dass** die Umformung des Steckerteils (20) stirnseitig beginnend mit als Führungen für die Schneidmesser dienenden Ausnehmungen (80) versehen ist, dass das Kabel (60) in einem vorgegebenen Abstand zur Stirnseite (70) der Umformung endet und dass in der Umformung zwischen der Ausnehmung (80) und dem freien Ende des Kabels (60) eine dünne isolierende Schicht (90) vorgesehen ist.

4. Steckverbindung nach den Ansprüchen 1 bis 3,
**dadurch gekennzeichnet,**
**dass** die Umformung integral mit einer elektrischen Isolierung (65) der elektrischen Leiter (63), vorzugsweise durch Umspritzen von Kunststoff verbunden ist.

5. Steckverbindung nach den Ansprüchen 1 bis 3,
**dadurch gekennzeichnet,**
**dass** die Umformung aus mindestens einem Körperteil besteht, dass auf die elektrische Isolierung (65) der elektrischen Leiter (63) aufgesetzt und durch eine Klemm- und/oder Quetsch- und/oder Schnappverbindung gehalten ist.

6. Steckverbindung nach den Ansprüchen 1 bis 5,
**dadurch gekennzeichnet,**
**dass** die Kontaktschneiden (50) der Kontaktierungsteile (40) des Steckerteiles (20) einen ersten gegenüber einer Einführungsachse abgewinkelten Bereich (150) und einen zweiten gegenüber der Einführungsachse abgewinkelten Bereich (160) aufweisen, der sich an den ersten Bereich (150) anschließt zum Anschneiden der die elektrischen Leiter (63) umhüllenden Isolierung (65) und gegebenenfalls eines kleinen Teils des elektrischen leitfähigen Bereichs der elektrischen Leiter (63), wobei beim Ineinanderschieben von Stecker- und Buchsenteil (20, 30) zunächst der zweite Bereich (160) der Kontaktschneiden (50) mit dem elektrischen Leiter (63) in Kontakt tritt und dass der Winkel des zweiten Bereiches (160) größer ist als der Winkel des ersten Bereiches (150).

7. Steckverbindung nach Anspruch 6,
**dadurch gekennzeichnet,**
**dass** der erste Bereich (150) einen Winkel von etwa 40° - 50° und der zweite Bereich einen Winkel von etwa 10° - 30° umfasst.

8. Steckverbindung nach einem der vorhergehenden Ansprüche,
**dadurch gekennzeichnet,**
**dass** der Kanal (35) des Buchsenteiles (30) in dem der Einführungsöffnung abgewandten Endbereich eine Öffnung (210) zum Ausbringen von sich im Kanal (35) angesammelten Verunreinigungen aufweist.

9. Steckverbindungen nach den Ansprüchen 1 bis 8,
**gekennzeichnet durch** deren Verwendung in einer medizinischen Körper-, fetalen Skalpoder ähnlichen Elektrode.

10. Steckverbindung nach Anspruch 9,
**dadurch gekennzeichnet,**
**dass** das Buchsenteil (30) Mittel (320) zum lösbaren Verbinden mit einem weiteren Bauteil (300) aufweist.

## Claims

1. A plug-in connector (10) for an electrical line (cable 60), more particularly for a number of medical applications enabling plug-in cycles, comprising a plug element and a socket element (30) with contacting elements (40) facing each other on either side of the socket element which project by contact edges (50) into a channel (35) of the socket element (30) which channel (35) is provided for enabling, complementary to the plug element, the reception of the torsion-locked plug element (20) which can be slid in and is used for contacting, which plug element (20) is arranged as an electrically insulating molding which envelops and positions the conductors (63) of the electrical line (60) which are surrounded by an insulation on the contacting side and has a stop (200), all this in such an arrangement that when the plug element (20) is slid into the channel (35) of the socket element (30) as far as the stop (200) the contact members (50) of the contacting elements (40), which members project into the channel (35) cut into the respective facing side of the insulation (65) of the cable (60) for the purpose of contacting conductors (63) of the cable (60) and assigned contacting element (40) of the socket element (30).

2. A plug-in connector as claimed in claim 1, **characterized in that** the plug-in element (20) has a cross-section serving as a torsion-locked device (75) for the purpose of inserting the plug element (20) and socket element (30) into each other in the correct position, which cross-section is not axially symmetrical, at least not relative to a central axis.

3. A plug-in connector as claimed in claims 1 and 2, **characterized in that** the molding of the plug element (20) starting at a head end has cut-outs (80) used as guides for the cutting knives, **in that** the cable (60) ends at a predefined distance from the head end (70) of the molding and **in that** a thin insulating layer (90) is provided in the molding between the cut-out (80) and the free end of the cable (60).

4. A plug-in connector as claimed in claims 1 to 3, **characterized in that** the molding is integrally connected with an electrical insulation (65) of the electrical conductors (63), preferably by molding of synthetic material.

5. A plug-in connector as claimed in one of the claims 1 to 3, **characterized in that** the molding comprises at least a body element which is arranged on the electrical insulation (65) of the electrical conductors (63) and/or retained by a clamp and/or crimp and/or snap connection.

6. A plug-in connector as claimed in one of the claims 1 to 5, **characterized in that** the contacting members (50) of the contact elements (40) of the plug element (20) have a first section (150) bent away from a lead-in axis and a second section (160) bent away from the lead-in axis, which second section is a continuation of the first section (150) for cutting the insulation (65) enveloping the electrical conductors (63) and a small part of the electrically conductive section of the electrical conductors (63) as desired in which, when plug and socket element (20,30) are slid into each other, first the second section (160) of the contacting members (50) comes into contact with the electrical conductor (63) and **in that** the angle of the second section (160) is larger than the angle of the first section (150).

7. A plug-in connector as claimed in claim 6, **characterized in that** the first section (150) has an angle of about 40°-50° and the second section an angle of about 10°-30°.

8. A plug-in connector as claimed in one of the preceding claims, **characterized in that** in the channel (35) of the socket element (30) in the final section facing away from the lead-in there is an opening (210) for removing impurities accumulated in the channel (35).

9. Plug-in connectors as claimed in the claims 1 to 8, **characterized by** the use in a medical body, fetal scalp or similar electrode.

10. A plug-in connector as claimed in claim 9, **characterized in that** the socket element (30) comprises means (320) for disengaging connection to a further component (300).

## Revendications

1. Connecteur (10) pour un conducteur électrique (câble 60), en particulier pour un nombre de cycles de connexion permettant des applications médicales, avec une pièce de fiche et une pièce de douille (30) avec des pièces de mise en contact (40) qui se font face sur des côtés opposés et pénètrent à l'aide de lames de contact (50) dans un canal (35) de la pièce de douille (30) de conception complémentaire à la pièce de fiche et permettant le logement de la pièce de fiche (20) servant à la mise en contact, à enfoncer à blocage de rotation, laquelle pièce de fiche (20) est conçue comme une gaine isolante électriquement qui entoure le conducteur (63) respectivement revêtu d'une isolation du conducteur électrique (60) en se positionnant côté mise en contact et présente une butée (200), tout cela dans un dispositif tel qu'en cas d'introduction de la pièce de fiche (20) délimitée par la butée (200) dans le canal (35) de la pièce de douille (30), les lames de contact (50) des pièces de mise en contact (40) pénétrant dans le canal (35) coupent dans la partie de l'isolation (65) du câble (60) respectivement tournée vers elles en vue de la mise en contact du conducteur (63) du câble (60) et de la pièce de mise en contact (40) affectée de la pièce de douille (30).

2. Connecteur selon la revendication 1, **caractérisé en ce que** la pièce de fiche (20) présente en vue de l'assemblage en position précise de la pièce de douille (30) et de la pièce de fiche (20) une section servant de blocage contre la rotation (75) qui est conçue pour ne pas être symétrique axialement du moins par rapport à un axe médian.

3. Connecteur selon l'une des revendications 1 et 2, **caractérisé en ce que** la gaine de la pièce de fiche (20) est dotée en commençant du côté face d'about d'évidements (80) servant de guidages pour les lames de découpage, que le câble (60) se termine à un intervalle préalablement déterminé par rapport à la face d'about (70) de la gaine et que, dans la gaine entre l'évidement (80) et l'extrémité libre du câble (60), il est prévu une fine couche isolante (90).

4. Connecteur selon l'une des revendications 1 à 3,
**caractérisé en ce que** la gaine est reliée intégralement à une isolation électrique (65) du conducteur électrique (63) de préférence par coulage par injection de plastique.

5. Connecteur selon l'une des revendications 1 à 3, **caractérisé en ce que** la gaine se compose d'au moins une partie de corps, qui est appliquée sur l'isolation électrique (65) du conducteur électrique (63) et est maintenue par une fixation par serrage et/ou coincement et/ou ressort.

6. Connecteur selon l'une des revendications 1 à 5, **caractérisé en ce que** les lames de contact (50) des pièces de mise en contact (40) de la pièce de fiche (20) présentent une première zone (150) coudée par rapport à un axe d'introduction et une deuxième zone (160) coudée par rapport à l'axe d'introduction qui est adjacente à la première zone (150) pour le découpage de l'isolation (65) entourant le conducteur électrique (63) et, éventuellement, d'une petite partie de la zone électriquement conductrice du conducteur électrique (63), la deuxième zone (160) des lames de contact (50) entrant d'abord en contact avec le conducteur électrique (63) en cas d'assemblage de la pièce de fiche et de la pièce de douille (30) et que l'angle de la deuxième zone (160) est supérieur à l'angle de la première zone (150).

7. Connecteur selon la revendication 6, **caractérisé en ce que** la première zone (150) forme un angle d'environ 40° - 50° et la deuxième zone un angle d'environ 10° - 30°.

8. Connecteur selon l'une des revendications précédentes, **caractérisé en ce que** le canal (35) de la pièce de douille (30) présente dans sa zone d'extrémité opposée à l'ouverture d'introduction une ouverture (210) pour l'évacuation d'impuretés qui se sont accumulées dans le canal (35).

9. Connecteurs selon l'une des revendications 1 à 8, **caractérisés par** leur mise en oeuvre dans une électrode médicale pour le corps, le cuir chevelu de foetus ou autre.

10. Connecteur selon la revendication 9, **caractérisé en ce que** la pièce de douille (30) présente des moyens (320) pour la fixation amovible à une autre pièce (300).
